# EUROPEAN PATENT APPLICATION

(11) **EP 4 275 742 A1**
(43) Date of publication of application: **15.11.2023**
(21) Application number: 22173019.5
(22) Date of filing: 12.05.2022
(51) Int. Cl.: A61N 5/10

(54) **COMPACT BEAM TRANSPORT SYSTEM FOR MULTI-ROOM PARTICLE THERAPY FACILITY**

(71) Applicant: PAUL SCHERRER INSTITUT, 5232 Villigen PSI (CH)
(72) Inventor: MARADIA, Vivek, Uitikon Waldegg 8142 (CH); LOMAX, Antony, 5210 Windisch (CH); PSOROULAS, Serena, 8050 Zürich (CH); MEER, David, 5200 Brugg (CH)
(74) Representative: Fischer, Michael

(57) **Abstract**

The present invention is related to a particle therapy apparatus used for particle therapy. More particularly, this invention relates to a compact rotating beamline to deliver the proton beam to multiple treatment rooms. The present invention shows a compact rotating beamline for particle therapy. This invention provides a way to reduce the beamline components while maximising the number of treatment rooms. As a result, it significantly reduces the investment cost of the multi-room facility and increases patient throughput.

## Description

The present invention relates to a charged particle therapy apparatus used for proton or ion beam radiation therapy.

Over the past three decades, proton therapy experienced remarkable developments and has become a credible option in radiotherapy to treat certain types of cancers. Proton therapy delivers less radiation to healthy tissues and organs, potentially resulting in less severe short and long-term side effects than standard radiation therapy, helping to improve survival rates and quality of life, and to reduce secondary cancer induction.

A typical proton therapy system can be divided into three different parts. The first part is an accelerator (cyclotron, synchrotron, or synchrocyclotron) which produces energetic charged particles. The second part is a transport section comprising a fixed beamline which transports the beam from the accelerator exit to the treatment room. The third part is a gantry which rotates the beamline about the horizontal axis, and allows to deliver the beam to the target from different directions.

In particular, and due to the magnetic rigidity of protons, proton gantries are typically large (~10m diameter) and therefore, the size and cost of proton therapy facilities are significantly higher compared to a conventional radiation therapy facility. The required investment for a proton center with 2 to 3 treatment rooms typically is around US$100 million. However, if cost was not an issue, proton therapy would widely replace conventional radiation therapy for several indications.

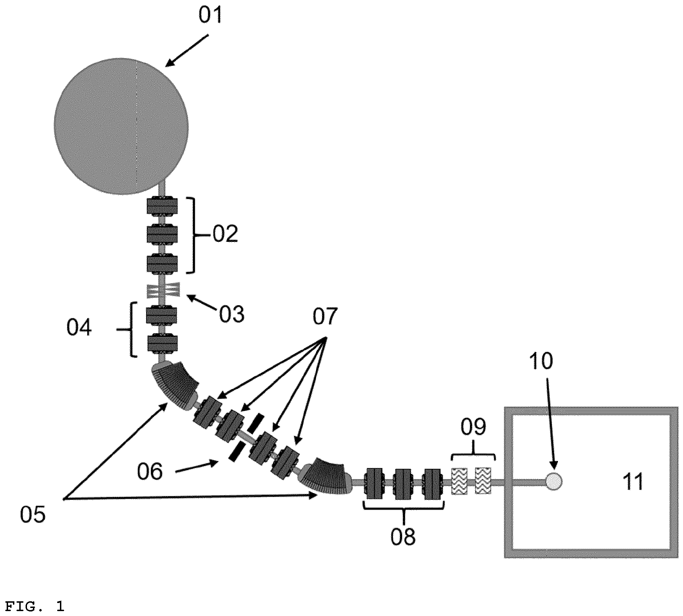

Recent studies showed that the majority of particle beam approaches can be realized with gantry-less delivery, where the patient is treated in an upright position and rotated in front of a static treatment beam. Therefore, the upright treatment approach has the potential to reduce the need for the gantry and reduce the size and cost of proton therapy. As shown in Fig 1, a typical cyclotron-based single room facility without gantry, requires a fixed beamline consisting of an energy degrader 3 for reducing the beam energy and an energy selection system (ESS) that reduces the energy spread induced by the degrader 3. The energy selection system is a part of the achromatic beamline which comprises two dipoles 5, four quadrupoles 7, and a movable slit system 6. After the ESS, two to three quadrupole magnets 8 are required to focus the beam at the patint location 10.

Therefore, a single-room, cyclotron-based fixed beamline facility requires at least 10 to 12 quadrupoles and at least 2 bending magnets. In addition, a multiroom cyclotron-based facility requires an additional achromatic bend 109 for each treatment room, increasing the beamline components.

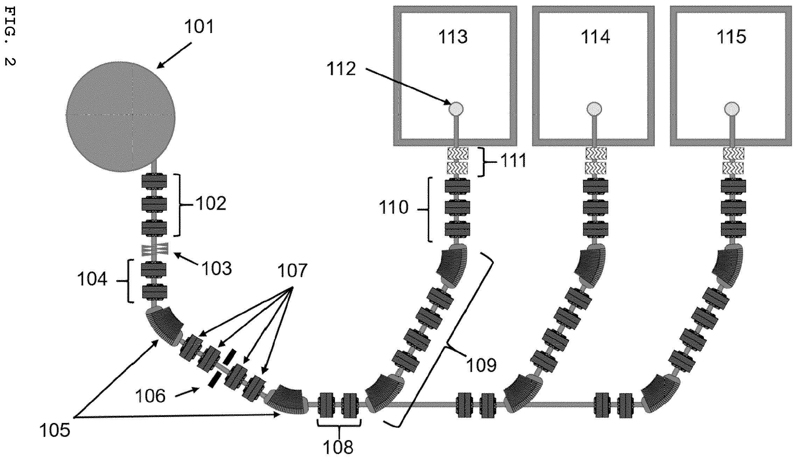

As shown in Fig 2, a three-room cyclotron-based fixed beamline facility may require 30 to 36 quadrupole and 8 dipole magnets. Every addition of a treatment room will add about 9 quadrupoles and 2 bending magnets to the beamline.

These beamline components require large power supplies and expensive external cooling systems. Additionally, for magnet cooling, it is necessary to use water cooling lines using deionized water which take significant space, leading to a large footprint and therefore cost.

In summary, such a multiroom, fixed beamline solution as shown in figure 2 will may also require significant building, shielding, and installation costs compared to a conventional radiation therapy facility. As such, a significant need exists to reduce the beamline components also for multiroom treatment facilities which utilize vertical positioning solutions.

It is therefore the objective of the present invention to provide a charged particle therapy apparatus used for proton or ion beam radiation therapy that enables to build a compact rotating beamline that can deliver the beam to multiple treatment rooms.

This objective is achieved according to the present invention by a particle beam transport system for the delivery of particle beam therapy to a plurality of treatment delivery rooms, comprising:
a) a particle beam source (201), such as an ion or proton accelerator,
b) a beam transport section comprising a number of beam bending and/or beam focusing magnets, an energy selection system and optionally scanning magnets for guiding the particle beam at a predefined energy and beam emittance to a patient therapy section,
c) a rotating mechanism bearing the beam transport section thereby enabling the beam transport section to be rotated clockwise or anti-clockwise around a central axis to deliver the beam into a radial direction as seen with respect to the central axis; and
d) said patient therapy section comprising the plurality of patient treatment rooms being disposed in a circular manner substantially within a common plane; said common plane being oriented substantially parallel to a plane given by the radial directions deductable from the rotation of the beam transport section.

The present invention therefore allows to realize a compact rotating beamline with minimum beamline components (magnets, power supplies) which can deliver beam to multiple treatment rooms for particle therapy. Since the present compact rotating beamline is enabled to rotate up to 360° about the vertical axis in both clockwise and anti-clockwise directions, the treatment delivery rooms can be built in a circular arrangement such that the rotating beamline can deliver beam to each treatment room. With the present invention, we can have a plurality of different treatment delivery rooms (for example up to 12) with an investment cost similar to a single treatment room facility. This will significantly increase patient throughput and reduce the cost of proton therapy.

In a preferred embodiment of the present invention, the rotating mechanism can be designed to rotate the beam transport section horizontally or vertically about the central axis.

In a further preferred embodiment of the present invention, the beam transport system is tilted with respect to an horizontal axis. This tilting can be slightly in the range of 5 to 25 degrees, i.e. 10 degrees, and allows for some kind of tumor treatments to spare healthy organs.

In a mechanically stable arrangement, the central axis can be oriented substantially vertically and the radial direction can be oriented substantially perpendicular to the central axis.

Preferred embodiments of the present invention are hereinafter described in more detail with reference to the attached drawings which depict in:
- Fig. 1: schematically a top view of an exemplary configuration of the single room cyclotron based proton therapy facility without gantry according to the prior art;
- Fig. 2: schematically a top view of an exemplary configuration of a three room cyclotron based proton therapy facility without gantry according to the prior art;
- Fig. 3: schematically a side view of a rotating beamline that transports the particle beam from the cyclotron to a plurality of the patient treatment rooms; and
- Fig. 4: schematically a top view of a rotating beamline and a plurality of 12 different treatment rooms.

The present invention will now be described in detail in relation to the appended drawings. However, it is evident that a person skilled in the art may conceive several equivalent embodiments or other ways of executing the present invention.

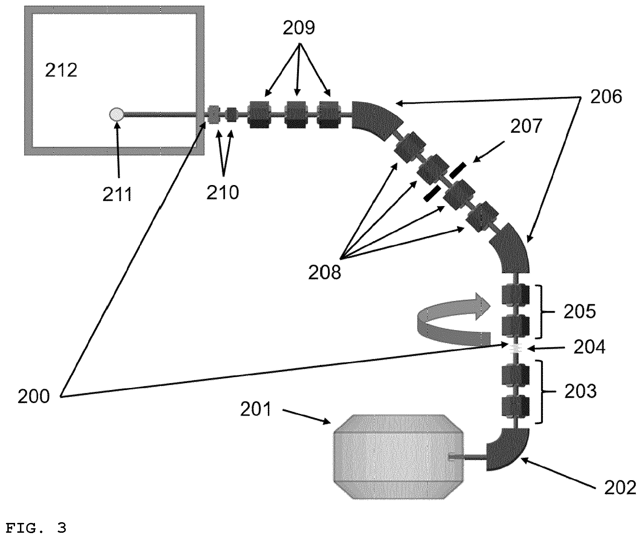

The present invention proposes a compact movable beamline with minimum beamline components to deliver a therapy beam to up to 12 different treatment rooms. Fig. 3 shows a side view of a preferred layout of such a movable beamline. A cyclotron 201 produces a high energy (250/230/200 MeV) proton beam. First, the high-energy proton beam 226 will pass through a 90° bending magnet 202 to bend the beam vertically. After that, a quadrupole doublet 203 focuses the high-enery proton beam at the center of an energy degrader 204 in form of a wedge degrader. The thickness of the wedge degrader is variable based on the requirement of beam energy for the patient treatment.

After the energy degrader 204, the downstream beamline forms a rotating beamline 200 which can rotate 360° (in a clockwise and anti-clockwise direction) about a central axis of the energy degrader 204. The first part of the rotating beamline 200 is a pair of quadrupole magnets 205 which focuses the beam at the entrance of an energy selection system ESS. The ESS is used to reduce the energy spread of the beam induced by the degrader 204 and comprises two 45° dipole magnets 206, four quadrupole magnets 208 and energy selection slits 207 to select the energy. After the ESS, a quadrupole triplet 209 is used to focus the beam at an isocenter 211 which represent the patient location. Two scanning magnets 210 are used to scan the beam over large target volumes.

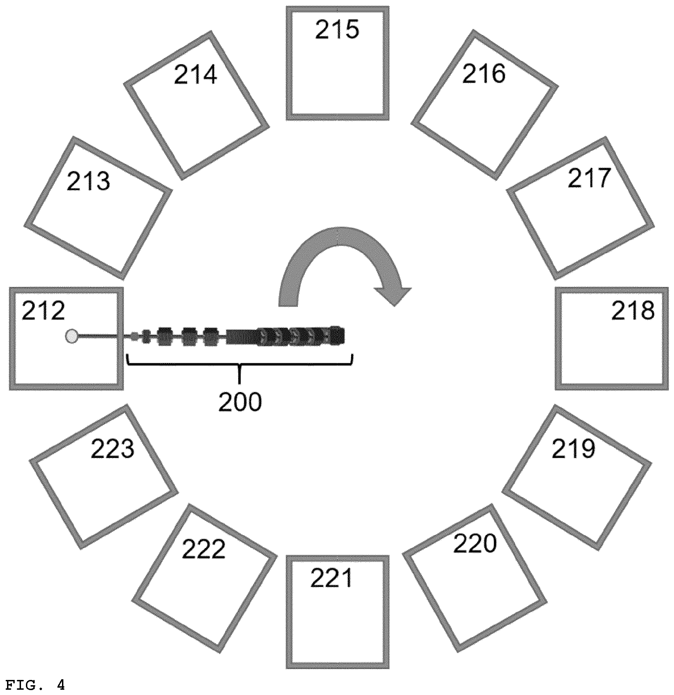

Fig. 4 shows a top view of the compact beamline 200 with 12 different treatment rooms 212 to 223. The dimensions of each treatment room are 4m x 4m. The rotating beamline 200 can rotate both clockwise and anti-clockwise around a central axis 225 to deliver the beam 226 into different treatment rooms 212 to 223 wherein all treatment rooms are equipped in the present example with upright treatment chairs. Said patient therapy section comprising the plurality of patient treatment rooms 212 to 223 are disposed in a circular manner substantially within a common plane (here the plain of the drawing). Said common plane is oriented substantially parallel to a plane given by the radial directions 227 deductable from the rotation of the beam transport section 200 around the central axis 225.

The weight of the rotating beamline 200 is significantly lower than that of a conventional gantry. It is therefore easy to rotate the beamline 200 from one to any another room within 30 seconds. With the present example of 12 treatment rooms 212 to 223, it would be possible to treat about 3,000 patients per year thereby increasing the patient throughput significantly compared to conventional gantry based facilities.

Conventional cyclotron-based fixed beamlines (as shown in Fig. 1 and Fig. 2) would require more than 100 quadrupole and 25 dipole magnets to deliver the beam in 12 different treatment rooms. Additionally, the footprint of the facility according to the state of the art is significantly higher. However, with the invention of the rotating beamline 200, the beam therapy can be delivered up to 12 different rooms with just 11 quadrupoles and 3 bending magnets.

The general mechanics of the rotating mechanism 224 is similar to the mechanism to rotate a complete gantry as currently in use in proton gantries but the advantage here is that this powerful rotating mechanism enables to share a single beam delivery system across the different treatment rooms. This will reduce the cost of the beamline components, cooling system, power supplies, and shielding will reduce significantly. Additionally, a 12-room facility has a footprint equivalent to two basketball fields only.

## Claims

1. A particle beam transport system for the delivery of particle beam therapy to a plurality of treatment delivery rooms, comprising:
a) a particle beam source (201), such as an ion- or proton accelerator,
b) a beam transport section (200) comprising a number of beam bending and/or beam focusing magnets (205, 208, 209), an energy selection system and optionally scanning magnets for guiding the particle beam at a predefined energy and beam emittance to a patient therapy section,
c) a rotating mechanism (224) bearing the beam transport section (200) thereby enabling the beam transport section (200) to be rotated clockwise or anti-clockwise around a central axis (225) to deliver the beam (226) into a radial direction (227) as seen with respect to the central axis (225);
d) said patient therapy section comprising the plurality of patient treatment rooms (212 to 223) being disposed in a circular manner substantially within a common plane; said common plane being oriented substantially parallel to a plane given by the radial directions (227) deductable from the rotation of the beam transport section (200).

2. The particle beam transport system according to claim 1, wherein the rotating mechanism (224) is designed to rotate the beam transport section (200) horizontally or vertically about the central axis (225).

3. The particle beam transport system according to claim 1 or 2, wherein the beam transport system (200) is tilted with respect to an horizontal axis (227).

4. The particle beam transport system according to any of the preceding claims wherein the central axis (225) is oriented substantially vertically and the radial direction (227) is oriented substantially perpendicular to the central axis (225).
